# EUROPEAN PATENT APPLICATION

(11) **EP 4 796 104 A1**
(43) Date of publication of application: **26.08.2026**
(21) Application number: 25382168.0
(22) Date of filing: 25.02.2025
(51) Int. Cl.: A61K 8/64, A61K 8/65, A61K 8/99, A61Q 19/08

(54) **COSMETIC COMPOSITION FOR SKIN REJUVENATION**

(71) Applicant: Germaine de Capuccini, SAU, 03801 Alcoi Alicante (ES)
(72) Inventor: GONZÁLVEZ ILLUECA, Ana, 03801 ALCOI (ES); CARBONELL RIPOLL, Carolina, 03801 ALCOI (ES)
(74) Representative: Torner, Juncosa I Associats, SL

(57) **Abstract**

The present disclosure relates to a cosmetic composition for skin rejuvenation comprising: fragments of type I collagen and type IV collagen, fibrillin microfibrils, and a marine extract. The present disclosure also relates to a method for the cosmetic, non-therapeutic treatment and/or care of the skin comprising the topical administration to a subject in need thereof of an effective amount of the cosmetic composition disclosed herein. The present disclosure also relates to a method for improving the state of the skin, preferably human skin, comprising applying topically to the skin of a subject in need thereof of an effective amount of the cosmetic composition disclosed herein. Furthermore, the present disclosure relates to the use of the cosmetic composition for the cosmetic, non-therapeutic treatment and/or care of the skin, mucous membranes, hair and/or nails.

## Description

### TECHNICAL FIELD

The present disclosure relates to a cosmetic composition for skin rejuvenation. The present disclosure also relates to a method for rejuvenating the skin and to uses of the compositions herein disclosed.

### BACKGROUND ART

The skin is the largest organ of the human body, forming an the outer layer of the body and being one of the most important organs. It consists of three morphologically differentiated layers, the epidermis, the dermis and the hypodermis, each of them performing different functions while interrelating with each other. Many of its physiological functions are determined by its stratified structure.

The epidermis is the outermost layer, primarily composed of keratinocytes, skin cells that produce keratin, providing a waterproof barrier. It also includes melanocytes, which produce melanin, responsible for skin color.

The dermis lies below the epidermis and contains connective tissue, blood vessels, nerve endings, hair follicles, and sweat glands. It provides structural support and elasticity. The main cell types in the dermis are fibroblasts, which are responsible for the synthesis of extracellular matrix components such as collagen, elastin and glycosaminoglycans.

The hypodermis is the deepest layer, made up of fat and connective tissue, which insulates the body and stores energy.

The skin plays an important role as a barrier that separates human beings from the external environment by regulating and preventing, among other things, water loss and the entry of microorganisms and xenobiotics.

The skin also has a great social impact since its appearance influences the social behavior of most individuals. Skin aging is a continuous process where the functional homeostasis of the skin is altered and different changes in its appearance appear.

Skin aging is a multifactorial process induced by both intrinsic and extrinsic causes. It results in phenotypic changes, as well as in the structure and functions of the components of the extracellular matrix such as collagen, elastin and glycosaminoglycans involved in providing strength, elasticity and hydration to the skin.

The morphological changes produced by aging are characterized by a thinner epidermis and dermis with a lower number of keratinocytes and fibroblasts. Thinning of the epidermis increases the aged appearance of the skin with the presence of fine wrinkles while compromising barrier function and increasing water loss. The thinning of the dermis due to a decrease in collagen and elastin fibers increases its fragility and results in a loss of firmness. In order to prevent skin aging and/or to improve the appearance of the skin, several solutions have been envisaged in the prior art.

For instance, there are solutions involving the paralysis of muscles, such as Botox or peptides that block acetylcholine.

Other solutions involve the use of products that stimulate the synthesis of collagen and elastin.

Currently there has also been a trend to use extracts of different species for cosmetic uses and treatments.

For instance, WO2018203000A1 discloses the use of an extract of *Nephelium lappaceum* to increase the firmness and/or elasticity of the skin and/or mucous membranes.

WO2010145008A1 disloses the use of skin care compositions comprising *Laminariacea* extract for treatment of skin aging signs.

However, there remains in the art a need for cosmetic compositions that are stable, provide with good values of elasticity/firmness and show skin rejuvenation properties, in particular high anti-wrinkle efficacy after application.

### SUMMARY OF THE DISCLOSURE

A first aspect of the present disclosure provides a cosmetic for skin rejuvenation comprising: fragments of type I collagen and type IV collagen, fibrillin microfibrils, and a marine extract.

A second aspect of the present disclosure provides a method for the cosmetic, non-therapeutic treatment and/or care of the skin comprising the topical administration to a subject in need thereof of an effective amount of the cosmetic composition according to the present disclosure.

A third aspect of the present disclosure provides a method for improving the state of the skin, preferably human skin, comprising applying topically to the skin of a subject in need thereof of an effective amount of the cosmetic composition according to the present disclosure.

A fourth aspect of the present disclosure relates to the use of the cosmetic composition according to the present disclosure for the cosmetic, non-therapeutic treatment and/or care of the skin, mucous membranes, hair and/or nails.

Other features of the present disclosure are disclosed in the detailed description of the disclosure.

### BRIEF DESCRIPTION OF THE FIGURES

The foregoing and other advantages and features will be more fully understood from the following detailed description of an embodiment with reference to the accompanying figures, to be taken in an illustrative and non-limitative manner, in which:

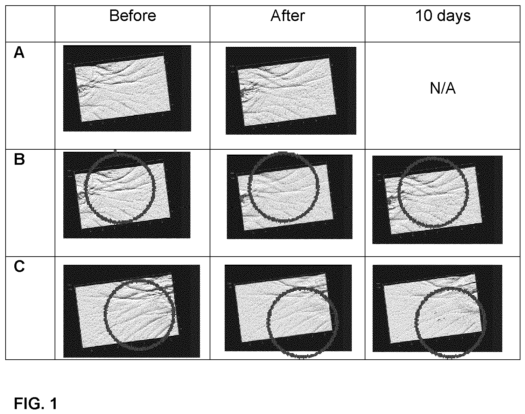

FIG. 1 shows the effect of the application of treatment A (blank control), treatment B (cosmetic composition according to the present disclosure without *Alteromonas* ferment extract) and treatment C (cosmetic composition according to the present disclosure).

### DETAILED DESCRIPTION OF THE DISCLOSURE

The foregoing and other advantages and features will be more fully understood from the following detailed description of the invention. The examples disclosed herein are to be taken in an illustrative and not limitative way.

Unless otherwise indicated, all percentages relating to the content of a component or to a collection of components of the composition of the present invention refer to the weight percentage with respect to the total weight of the composition.

In the context of the present disclosure, the expressions "according to the disclosure", "preparation according to the disclosure", "according to the present disclosure", etc. always refer to the preparations, processes and uses according to the disclosure, i.e. also to preparations in which the uses according to the disclosure are realized as well as preparations with which the inventive method is realized.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The present disclosure relates, as defined, to a cosmetic composition for skin rejuvenation comprising: fragments of type I collagen and type IV collagen, fibrillin microfibrils and a marine extract.

In the context of the present disclosure, the expression "fragments of type I collagen and type IV collagen" refers to smaller pieces or portions, i.e., small proteins or peptides, of the two types of collagen proteins, type I and type IV collagen.

Type I collagen is the most abundant form of collagen in the body and is found in bones, skin, tendons, and other connective tissues. It provides structural support and strength.

Type IV collagen is primarily found in the basement membranes, which are thin layers of extracellular matrix that support cells, particularly in tissues like the kidneys, lungs, and blood vessels.

In the context of the present disclosure, the expression "fibrillin microfibrils" refers to small, thread-like structures made primarily of fibrillin, a glycoprotein that plays a crucial role in the formation of the extracellular matrix, which provides structural support to tissues.

Fibrillin is a large, extracellular matrix protein that forms the core of microfibrils. It is composed of a series of repeating domains, and its primary function is to provide strength and elasticity to connective tissues. Fibrillin is essential for the formation of structures like elastic fibers in tissues such as skin, blood vessels, and lungs.

Microfibrils are fine, fibrous structures that are often too small to be seen without a microscope. These are made up of fibrillin and other components like elastin in certain tissues, and they form a scaffold that helps to organize and support the larger structures in the extracellular matrix. Microfibrils are important in maintaining the integrity and elasticity of tissues, particularly in areas where flexibility is needed, such as in the walls of blood vessels.

In the context of the present disclosure, the expression "marine extract" refers to any substance that is derived from marine organisms, such as fish, algae, seaweed, mollusks, corals, or even microorganisms found in the ocean. These extracts are often rich in bioactive compounds like proteins, peptides, lipids, minerals, and antioxidants, which are valued for their potential health, cosmetic, or pharmaceutical benefits.

In particular, the cosmetic composition according to the present disclosure may comprise a marine extract which is a marine bacteria extract. Even more particular, the marine bacteria are from the *Alteromonas* genus.

The marine bacteria extract may also preferably be a ferment extract from a bacteria. Even more particularly, the marine bacteria extract may be a ferment extract from a bacteria found in a marine organism. The marine organism is preferably a jellyfish.

A "ferment extract" is understood herein as a substance that is derived from a fermentation process, in which microorganisms like bacteria, yeast, or fungi break down organic materials (such as sugars or proteins) into simpler compounds. These extracts contain a variety of bioactive compounds, including enzymes, amino acids, peptides, vitamins, and other metabolites that are produced during fermentation.

In particular, the ferment extract from *Alteromonas* may be a fermentation product of an extract of *Alteromonas macleodii.*

The marine ferment extract may be preferably produced by a biofermentation process, that is, a biological process in which microorganisms break down organic materials in an anaerobic or aerobic environment to produce desired products, typically involving the steps of inoculation, fermentation, product formation and harvesting.

In particular, the marine extract in the present disclosure is the INCI product known as Alteromonas Ferment Extract.

In the cosmetic composition according to the present disclosure, the fragments of type I collagen and type IV collagen are preferably a combination of hexapeptide-40 and sh-polypeptide-47. Even more preferably, the fragments of type I collagen and type IV collagen in the present disclosure correspond to the INCI product known as Nicotiana benthamiana hexapeptide-40 sh-polypeptide-47.

In the cosmetic composition according to the present disclosure, the fibrillin microfibrils may preferably be a mixture of polypeptides mainly based on arginine and lysine. In particular, the fibrillin microfibrils may be the arginine/lysine polypeptide with CAS number 31014-78-5.

The cosmetic composition according to the present disclosure may preferably comprise the fragments of type I collagen and type IV collagen in an amount of 0,00001% and 0,0005% by weight with respect to the total weight of the cosmetic composition, i.e. 0,01 to 5 ppm.

The cosmetic composition according to the present disclosure may preferably comprise the the fibrillin microfibrils in an amount of 0,00001% and 0,0005% by weight with respect to the total weight of the cosmetic composition, i.e. 0,01 to 5 ppm.

The cosmetic composition according to the present disclosure may preferably comprise the the marine extract in an amount of 0,01% and 5% by weight with respect to the total weight of the cosmetic composition, more preferably between 0,01% and 2%.

The cosmetic composition according to the present disclosure may further at least one cosmetically acceptable excipient, adjuvant and/or ingredient.

In particular, the cosmetically acceptable excipient, adjuvant and/or ingredient might be an emollient.

In particular, the cosmetically acceptable excipient, adjuvant and/or ingredient might be selected from the list consisting of glycerin, caprylyl glycol, propylene glycol, and combinations thereof.

The cosmetic composition according to the present invention may further comprise hyaluronic acid.

The cosmetic compositions may be applied to mammalian keratinous tissue, in particular to human skin. The cosmetic compositions may take various forms. For example, some non-limiting examples of forms include solutions, suspensions, lotions, creams, gels, toners, sticks, pencils, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, cosmetics (e.g. foundations, eye liners, eye shadows), and the like.

The present disclosure also relates to a method for the cosmetic, non-therapeutic treatment and/or care of the skin comprising the topical administration to a subject in need thereof of an effective amount of the cosmetic composition as disclosed herein.

The present disclosure relates also to a method for rejuvenating the skin, characterized in that it comprises the topical application of the cosmetic composition herein disclosed.

Moreover, the present disclosure also relates to a cosmetic method for improving the state of the skin, preferably human skin, comprising applying topically to the skin of a subject in need thereof of an effective amount of the cosmetic composition as disclosed herein.

"Improve skin condition" or "improving skin condition" means effecting a visually and/or tactilely perceptible positive change, or benefit, in skin appearance and feel. Benefits that may be provided include, but are not limited to, one or more of the following: reducing the appearance of wrinkles, coarse deep lines, fine lines, crevices, bumps, and large pores; thickening of keratinous tissue (e.g., building the epidermis and/or dermis and/or sub-dermal layers of the skin, and where applicable, the keratinous layers of the nail and hair shaft, to reduce skin, hair, or nail atrophy); increasing the convolution of the dermal-epidermal border (also known as the rete ridges); skin lightening; preventing loss of skin or hair elasticity, for example, due to loss, damage and/or inactivation of functional skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by hyperpigmentation, etc.

The cosmetic method may preferably be characterized in that the method for improving the state of the skin is selected from reducing the wrinkles on the skin.

Many regimes exist for the application of the cosmetic composition. The cosmetic composition may be applied as needed and/or at least once a day, twice a day, or on a more frequent daily basis, during a treatment period. Non-limiting examples of the treatment periods may be between about 1 week and about 12 weeks, between about 4 weeks and about 12 weeks, and/or between about 4 weeks and about 8 weeks. In another example, the treatment period may extend over multiple months (i.e., 3-12 months) or multiple years. In another example, the cosmetic composition may be applied least once a day during a treatment period of at least about 4 weeks or at least about 8 weeks. In another example, the cosmetic composition may be applied twice a day during a treatment period of at least about 4 weeks or 8 weeks. In another example, the cosmetic composition can also be applied to at least one skin surface area at least once per day, twice per day, or three times per day for a period of 7, 14, 21, or 28 days or more. When applied twice daily, the first and second applications may be separated by at least 1 to about 12 hours. The cosmetic composition may be also applied in the morning and/or in the evening before bed. The treatment period should be a sufficient time to provide an improvement in the skin surface but need not be so. Preferably, in the cosmetic method the composition may be applied at least once a day for at least 10 days. Even more preferably, the composition may be applied at least twice or three times a day for at least 7 days, at least 10 days, at least 15 days or at least 21 days.

The present disclosure also relates to the use of the cosmetic composition for the cosmetic, non-therapeutic treatment and/or care of the skin, mucous membranes, hair and/or nails.

In particular, the use of the cosmetic composition may improve the appearance of facial skin.

The cosmetic composition according to the present disclosure comprises the different components herein described. The effect of the components in the skin have a synergistic effect of the different effects each of the ingredients have.

In particular, the marine extract in the cosmetic composition according to the present invention offers a smoother and more progressive modulation of muscle contraction. The present cosmetic composition has an effect that is not limited to blocking the neuromuscular signal, but modulates communication at multiple levels (presynaptic, postsynaptic and neuromuscular), allowing results without facial stiffness.

The fragments of type I and type IV collagen reconstitute collagen structure in the skin, improving not only the firmness but also the elasticity, contributing to the reduction of static wrinkles.

The fibrillin microfibrils restore skin elasticity, creating a "spring effect" that improves the resistance of the skin to aging.

The combination of the different components in the cosmetic composition as disclosed herein increase the collagen production and natural hyaluronic acid, have moisturizing and filling benefits and reinforces the cutaneous barrier.

The cosmetic composition as disclosed herein has the capability of reducing dynamic wrinkles (caused by facial expressions) and static wrinkles (present at rest), improving skin elasticity and firmness, increasing hydration and cell regeneration, and protecting the skin from environmental factors. In addition, it offers a rejuvenating effect, providing smoother, brighter and more resilient skin, without altering facial expressions.

The economic importance of this invention may be considerable, as it addresses one of the largest segments of the cosmetics industry: anti-aging. By offering an effective and non-invasive solution that combines advanced biofermentation technology, neuromuscular modulation and skin regeneration, it has great market potential. In addition, the growing demand for sustainable and biotechnological ingredients positions this product as highly attractive for both consumers and high-end brands.

### EXAMPLES

### Example 1: Cosmetic ingredients

The cosmetic composition according to the present disclosure may preferably comprise a combination of three ingredients that synergically act improving the appearance of skin.

The cosmetic composition may be preferably formulated from the following ingredients:

### 1- Water soluble Alteromonas ferment extract

This ingredient is obtained by fermentation of an extract from *Alteromonas* combined with other additivies. The ingredient may preferably comprise water (CAS number 7732-18-5), glycerin (CAS number 56-81-5), caprylyl glycol (CAS number 1117-86-8) and the *Altermomonas* ferment extract. The amount of the components in this ingredient may be about 33%, 65%, 1% and 1% by weight with respect to the total amount of ingredient, respectively.

### 2- Type I and type IV collagen fragments

Type I and type IV collagen fragments are obtained from vegetal biofactories, the ingredient being a combination of those fragments together with other additives. This ingredient may preferably comprise water (CAS number 7732-18-5), glycerin (CAS number 56-81-5), pentylene glycol (CAS number 5343-92-0) and collagen amino acids, in particular type I and type IV collagen fragments, even more particularly Nicotiana benthamiana hexapeptide-40 sh-polypeptide-47. The amount of the components in this ingredient may be about 32%, 65%, 2,3% and 0,0001% by weight with respect to the total amount of the ingredient, respectively.

### 3- Fibrillin microfibrils

This ingredient may preferably comprise water (CAS number 7732-18-5), glycerin (CAS number 56-81-5), and fibrillin microfibrils, those fibrillin microfibrils being arginine/lysine polypeptide (CAS number 31014-78-5). The amount of the components in this ingredient may be about 23%, 77% and 0,0001% by weight with respect to the total amount of the ingredient, respectively.

### Example 2: Cosmetic composition

To obtain the cosmetic composition as disclosed herein, the three ingredients above (1, 2 and 3) are mixed together in liquid form at a constant temperature, preferably about 25°C, ensuring the integrity of the bioactive compounds.

The pH of the mixture is adjusted and maintained in a range of between 5 and 7,5, being this pH the optimum range for the stability of the ingredients and the compatibility of the cosmetic composition with the human skin.

The mixture is homogenized until a convenient viscosity and product appearance is obtained. The cosmetic composition may have a colorless to light yellow aspect, is uniform and has cosmetic quality.

A preferred composition of the cosmetic composition as disclosed herein has the following components: glycerin, water, pentylene glycol, caprylyl glycol, *Alteromonas* ferment extract, collagen amino acids, and arginine/lysine polypeptide. The amount of *Alteromonas* ferment extract in this composition may preferably be between 0,01 and 2% by weight with respect to the total amount of the composition, while the amount of collagen amino acids and the arginine/lysine polypeptide may be between 0,00001% and 0,0005% by weight with respect to the total amount of the composition i.e. between 0,01 ppm and 5 ppm.

### Example 3: Efficacy studies

Three male and female volunteers aged between 30 and 55 underwent a professional treatment consisting of the application of 3 products, applied layer by layer:
- Point treatment, wherein the cosmetic composition is applied locally with vigorous movements
- Massage essence, wherein the cosmetic composition is applied to the entire face with a manual massage
- Facial mask, wherein the cosmetic composition is applied to the face and left for 15 to 20 minutes.

The treatment involved the cosmetic composition herein disclosed in an amount of between 4.5 to 9% by weight with respect to the total product applied to the skin.

Three-dimensional photography was taken with high resolution PRIMOS, based on a digital strip projection technique that allows for high-precision relocation of areas before and after each treatment with micrometric precision. The effectiveness of the treatment on expression wrinkles in the periocular area is determined.

To verify effectiveness, photographs were taken three times before treatment, post-treatment and 10 days after maintenance treatment at home consisting of facial serum, facial cream and eye contour.

Three different treatments were carried out:
Treatment A - blank control, i.e., a cosmetic composition not comprising type I collagen and type IV collagen fragments, fibrillin microfibrils, nor a marine extract.
Treatment B - cosmetic composition as disclosed herein but without the presence of *Alteromonas* ferment extract
Treatment C - cosmetic composition as disclosed herein (comprising *Alteromonas* ferment extract)

All volunteers underwent the treatment and 3D photographs were taken with the PRIMOS High Resolution equipment before the treatment, after the treatment and 10 days after undergoing the treatment at home.

The volunteer underwent treatment A and 3D photographs were taken before and after the treatment and the results were analyzed (see FIG. 1).

As shown in FIG. 1, no evolution is observed in the expression lines beyond the manual activation of the base treatment, so it was decided to continue applying treatment B and C to the volunteer.

Treatments B and C were performed on the same volunteer, applying treatment B to the left side of the face and treatment C to the right side of the face of the volunteer.

3D photographs were taken at time 0 (before the treatment), after the treatment and after 10 days of treatment with the corresponding home treatment.

As shown in FIG. 1, when comparing the three treatments A, B and C, the following evidence can be derived:
- The application of treatment A does not present an improvement on the skin beyond the manual topical application of a base cosmetic formulation
- When proceeding with treatment B, immediate results are observed after treatment, which are gradually lost over the 10 days that pass until the new 3D photographs are taken
- When proceeding with treatment C, immediate results are observed that are maintained, even improving over time as observed in the 3D photograph taken 10 days after the treatment.

These results show that the cosmetic composition according to the invention has a booster effect and effectively removes wrinkles in the skin. The presence of *Alteromonas* ferment extract in the cosmetic composition enables the modulation of the muscle contractions that are characteristic of facial expressions. Its differentiating effect on dynamic wrinkles means that the results are maintained and even improved after 10 days of treatment application using home products containing the cosmetic composition as disclosed herein.

## Claims

1. A cosmetic composition for skin rejuvenation comprising:
fragments of type I collagen and type IV collagen,
fibrillin microfibrils, and
a marine extract.

2. The cosmetic composition according to claim 1, wherein the marine extract is a marine bacteria extract.

3. The cosmetic composition according to claims 1 or 2, wherein the marine bacteria are from the *Alteromonas* genus.

4. The cosmetic composition according to any one of the previous claims, wherein the marine extract is a ferment extract.

5. The cosmetic composition according to any one of the previous claims, wherein the fragments of type I collagen and type IV collagen are a combination of hexapeptide-40 and sh-polypeptide-47.

6. The cosmetic composition according to any one of the previous claims, wherein the fragments of type I collagen and type IV collagen are in an amount of between 0,00001% and 0,0005% by weight with respect to the total weight of the cosmetic composition and/or the fibrillin microfibrils are in an amount of between 0,00001% and 0,0005% by weight with respect to the total weight of the cosmetic composition.

7. The cosmetic composition according to any one of the previous claims, wherein the marine extract is in an amount of between 0,01% and 2% by weight with respect to the total weight of the cosmetic composition.

8. The cosmetic composition according to any one of the previous claims, further comprising at least one cosmetically acceptable excipient, adjuvant and/or ingredient.

9. The cosmetic composition according to any one of the previous claims, further comprising hyaluronic acid.

10. A method for the cosmetic, non-therapeutic treatment and/or care of the skin comprising the topical administration to a subject in need thereof of an effective amount of the cosmetic composition according to any one of claims 1 to 9.

11. A cosmetic method for improving the state of the skin, preferably human skin, comprising applying topically to the skin of a subject in need thereof of an effective amount of the cosmetic composition according to any one of claims 1 to 9.

12. The cosmetic method according to claim 11, **characterized in that** the method for improving the state of the skin is selected from reducing the wrinkles on the skin.

13. The cosmetic method according to claim 11 or 12, **characterized in that** the cosmetic composition according to any one of claims 1 to 9 is applied at least once a day for at least 10 days.

14. Use of the cosmetic composition according to any one of the claims 1 to 9 for the cosmetic, non-therapeutic treatment and/or care of the skin, mucous membranes, hair and/or nails.

15. The use according to claim 14, wherein the cosmetic composition improves the appearance of facial skin.
